Europäisches Patentamt

**European Patent Office**

**Office européen des brevets**

(11) Veröffentlichungsnummer: **0 121 851**
**B1**

(12) **EUROPÄISCHE PATENTSCHRIFT**

(45) Veröffentlichungstag der Patentschrift:
13.01.88

(51) Int. Cl.⁴: **C 12 N 11/04**, C 02 F 3/00,
B 01 D 53/00

(21) Anmeldenummer: **84103298.0**

(22) Anmeldetag: **26.03.84**

(54) Biologisch aktive Zusammensetzung zur Abwasser- und Abluftreinigung.

(30) Priorität: **08.04.83 DE 3312578**

(43) Veröffentlichungstag der Anmeldung:
**17.10.84 Patentblatt 84/42**

(45) Bekanntmachung des Hinweises auf die Patenterteilung:
**13.01.88 Patentblatt 88/2**

(84) Benannte Vertragsstaaten:
**AT DE FR GB SE**

(56) Entgegenhaltungen:
**EP-A-0 089 165**
**DE-B-2 633 259**
**US-A-4 195 129**

(73) Patentinhaber: **BAYER AG, Konzernverwaltung RP
Patentabteilung, D-5090 Leverkusen 1 Bayerwerk
(DE)**

(72) Erfinder: **Baumgarten, Jörg, Dr., Henselweg 13,
D-5600 Wuppertal 1 (DE)**
Erfinder: **Frommer, Werner, Prof. Dr.,
Claudiusweg 17, D-5600 Wuppertal 1 (DE)**
Erfinder: **Mann, Theo, Dr., Blumenstrasse 18,
D-4018 Langenfeld (DE)**
Erfinder: **Pascik, Imre, Dr., Wilmersdorfer Strasse
6, D-4019 Monheim (DE)**
Erfinder: **Rast, Hans-Georg, Dr., Dierath 50, D-5653
Leichlingen (DE)**
Erfinder: **Schäpel, Dietmar, Dr., Johanniterstrasse
15, D-5000 Köln 80 (DE)**

EP 0 121 851 B1

## Beschreibung

Die Erfindung betrifft biologisch aktive Zusammensetzungen auf Basis von feinteiliger, oberflächenaktiver Kohle, Polymere mit kationischen Gruppen und wachstumsfähige Zellen enthaltender Hydrogele sowie ein Verfahren zu ihrer Herstellung durch Umsetzung von Isocyanat-Prepolymeren mit wäßrigen Suspensionen, die oberflächenaktive Kohle und Polymere mit kationischen Gruppen enthalten, wobei die wachstumsfähigen Zellen zusammen mit der wäßrigen Suspensionen direkt immobilisiert oder in einem zweiten Schritt dem zunächst erhaltenen Hydrogel zugefügt werden.

Die Kombination von oberflächenaktiven Feststoffen mit Mikroorganismen, um deren Aktivität bei Biokonversionsprozessen zu erhöhen, ist bekannt. Beispielsweise werden gemäß FR-A-2 233 334 zu Enzym-Suspensionen Ionenaustauscharze zugefügt. In den DE-A-2 633 259 und DE-A-2 703 834 wird die Adsorption von Zellen an z. B. Al₂O₃, Bentoniten und SiO₂ und deren nachfolgende Einbettung in Polyacrylaten beschrieben.

Die Einbettung von Zellen in z. B. Polyacrylat- oder Polyurethangelen zur verbesserten Handhabung der Zellen bei Biokonversionsprozessen ist bekannt. Die DE-A-2 629 692 beschreibt die Einlagerung in photohärtbares Harz, z. B. in Polyurethan, das photovernetzbare Acrylatdoppelbindungen enthält. Tanaka et al. beschreiben in European Journal of Applied Microbiology and Biotechnology 7, (1979) Seiten 351 bis 354 ein Verfahren, um vollständige Zellen in Polyurethan-Hydrogel einzubetten. Die nicht wachstumsfähigen Zellen weisen im immobilisierten Zustand enzymatische Aktivität bei Biokonversionsprozessen auf. In der DE-A-2 929 872 ist die Einbettung von gegebenenfalls wachstumsfähigen Zellen in Polyurethan-Hydrogelen beschrieben.

Die bisher bekannten Kombinationen von oberflächenaktiven Stoffen und Zellen, die gegebenenfalls auch in Gele eingebettet waren, eigneten sich nicht, um Abwasser oder Abluft einfach und effektiv zu reinigen.

Es wurde nun gefunden, daß die Abbauleistung von in Hydrogelen immobilisierten Zellen für Abwasser und Abluft sowie deren Handhabung bei Reinigungsprozessen verbessert werden kann, wenn man wachstumsfähige Zellen in Gegenwart von oberflächenaktiver Kohle und Polymeren mit kationischen Gruppen mittels Polyurethan-Hydrogelen, die durch Umsetzung von Isocyanat-Prepolymeren mit Wasser erhalten werden, immobilisiert.

Die Erfindung betrifft daher Zellen mit enzymatischer Aktivität enthaltende Trägermaterialien auf Basis von oberflächenaktive Kohle, Polymere mit kationischen Gruppen und Zellen mit enzymatischer Aktivität enthaltender Polyurethan-Hydrogele, die dadurch gekennzeichnet sind, daß die mittels Polyurethan-Hydrogelen immobilisierten Zellen wachstumsfähige Zellen sind.

Die Erfindung betrifft ferner ein Verfahren zur Herstellung von biologisch aktiven Zusammensetzungen auf Basis von oberflächenaktiver Kohle, Polymere mit kationischen Gruppen und wachstumsfähige Zellen enthaltender Hydrogele; das Verfahren ist dadurch gekennzeichnet, daß man Gemische aus oberflächenaktiver Kohle und Polymeren mit kationischen Gruppen in Polyurethan-Hydrogele eingelt, wobei vorzugsweise wachstumsfähige Zellen direkt mit eingebettet werden oder in einem nachfolgenden Schritt dem erhaltenen Gel zugefügt werden.

Beim Herstellungsverfahren können gegebenenfalls Di- und/oder Polyamine mitverwendet werden, wobei die Verwendung von Wasser als alleinigem Kettenverlängerungsmittel bevorzugt ist.

Als in den erfindungsgemäßen Trägermaterialien einsetzbare Zellen können die in Faul- und Belebtschlämmen von Kläranlagen vorkommenden Zellen und/oder für die Metabolisierung spezieller Substanzen adaptierte wachstumsfähige Zellen verwendet werden. Letztere Zellen stellen zur Aktivitätserhöhung speziell adaptierte herkömmliche Belebtschlamm-Organismen dar. Solche Mikroorganismen sind z. B. in Appl. Environ Microbiol., Vol. 42 (1981), p. 44-55, (1981), DE-A-3 046 686 oder DE-A-3 225 885 beschrieben.

Der Gehalt an Zellsubstanz, bezogen auf Trockenmasse, in den erfindungsgemäßen Zusammensetzungen beträgt 0,3 bis 10, gegebenenfalls 15 Gew.-%, vorzugsweise 0,5 bis 10 Gew.-%, besonders bevorzugt 0,8 bis 5 Gew.-%, bezogen auf das Gesamtgewicht des Reaktionsgemisches zur Herstellung der Zusammensetzung.

Als Polymere mit kationischen Gruppen kommen erfindungsgemäß beispielsweise handelsübliche Ionenaustauscharze mit kationischen Gruppen oder sonstige Polymere mit positive Ladungen aufweisende Stickstoffatome enthaltenden Strukturen in Betracht, wie zum Beispiel:

Polyaminocarbonsäureester mit kationischen Gruppen,

Polyacrylamide mit kationischen Gruppen, Polyethylenimine mit kationischen Gruppen, Copolymere aus Acrylnitril, Styrol und Dimethylaminoethylmethacrylat mit kationischen Gruppen,

Kondensationsprodukte aus Diethylentriamin und Maleinsäureanhydrid mit kationischen Gruppen, Copolymere aus Isobutylen und Maleinsäureanhydrid, und anschließender Imidisierung mit speziellen Diaminen, mit kationischen Gruppen.

Diese Substanzen werden als wäßrige Dispersionen in die erfindungsgemäßen Zusammensetzungen eingebettet.

Der Gehalt an Polymeren mit kationischen Gruppen in den erfindungsgemäßen Zusammensetzungen beträgt 0,2 - 20 Gew.-%, vorzugsweise 0,5 - 15 Gew.-%, besonders bevorzugt 1 - 10 Gew.-%, bezogen auf das Gesamtgewicht des Reaktionsgemisches zur Herstellung der Zusammensetzung.

Unter feinteiliger, oberflächenaktiver Kohle im Sinne dieser Erfindung sind Aktivkohle oder verwandte Substanzen bzw. Vorprodukte für Aktivkohle, die bereits Aktivkohleeigenschaften wie hohe spezifische Oberläche, Porosität und Ausbildung von Oberflächenladungen zeigen, zu verstehen. Solche sind z. B. durch Pyrolyse nachbehandelte natürliche Kohlen, pyrolysiertes Knochenmehl, Ruß usw. Wesentliches Erfordernis ist eine spezifische Oberfläche nach BET von oberhalb 50, vorzugsweise oberhalb 100 $\frac{m^2}{g}$.

Erfindungsgemäß verwendbare Typen von Aktivkohle sind solche, die in großtechnischen Produktionsverfahren anfallen, weiterhin solche, die durch geeignete Zusätze neutralisiert worden sind und darüber hinaus gegebenenfalls einer sogenannten Entaschung unterworfen worden sind.

Die Kohletypen können einzeln oder in Abmischungen verwendet werden.

Die Korngröße der oberflächenaktiven Kohle-Typen kann zwischen 0,5 und 1000 μm liegen. Der Gehalt an diesen Substanzen beträgt 0,5 bis 40 Gew.-%, vorzugsweise 0,5 bis 30 Gew.-%, besonders bevorzugt bis 20 Gew.-%, bezogen auf das Gesamtgewicht des Reaktionsgemisches.

Die in den erfindungsgemäßen Trägermaterialien enthaltenen kovalent vernetzten Polyurethan-Hydrogele sind durch Umsetzung von NCO-Prepolymeren bzw. -Semiprepolymeren mit Wasser, in dem sich gegebenenfalls Di- oder Polyamine als Kettenverlängerungsmittel bzw. Vernetzer befinden, erhältlich. Derartige Gele sind z. B. in der DE-A-2 347 299 und in der DE-A-2 521 277 beschrieben. Die Prepolymere und Semiprepolymere weisen Isocyanatendgruppen auf und werden in an sich bekannter Weise durch Umsetzung von Polyethern, die mindestens 30 Gew.-% -CH$_2$.CH$_2$.O-Einheiten ("Ethylenoxid-Einheiten") enthalten, mit einer überschüssigen Menge an Di- und/oder Polyisocyanat hergestellt. Bei dieser Umsetzung wird die Menge an Di- und/oder Polyisocyanat vorzugsweise so bemessen, daß das NCO/OH-Verhältnis in Abhängigkeit von den gewünschten Eigenschaften des herzustellenden Prepolymer-Typs 2 bis 10 beträgt. Der Gehalt des Prepolymers bzw. Semiprepolymers an Isocyanatgruppen beträgt vorteilhaft 1 bis 15 Gew.-%, vorzugsweise 2 bis 10 Gew.-%, besonders bevorzugt 2,5 bis 4,5 Gew.-%, bezogen auf das Gewicht des Prepolymers bzw. Semiprepolymers.

Erfindungsgemäß besonders bevorzugt sind Isocyanat-Prepolymere, die durch geeignete Produktionsschritte, wie zum Beispiel Dünnschicht-Distillation, von monomeren Di- bzw. Polyisocyanaten soweit befreit worden sind, daß deren Monomerengehalt unter 1 Gew.-%, bevorzugt unter 0,5 Gew.-%, liegt, wobei der Isocyanatgehalt 1,5 bis 4 Gew.-%, bezogen auf das gedünnschichtete Prepolymer, beträgt. Bevorzugte Isocyanatprepolymere sind daher solche auf Basis von Toluylendiisocyanaten.

Ausgangsmaterial für die NCO-Prepolymere bzw. Semiprepolymere sind mindestens zwei aktive Wasserstoffatome aufweisende Polyoxyalkylenether mit einem Molekulargewicht von 500 bis 10 000, vorzugsweise 2 000 bis 8 000, die mindestens 30 Gew.-% an Ethylenoxidgruppen in Form von Oxyethylengruppen [O.CH$_2$.CH$_2$], bezogen auf das Gewicht des Polyethers, neben vorzugsweise Oxypropylengruppen enthalten. Derartige Polyether werden durch Umsetzung von Verbindungen mit reaktionsfähigen Wasserstoffatomen, z. B. Di- oder Polyalkoholen, Di- oder Polyphenolen, aliphatischen oder aromatischen Di- oder Polyaminen, mit Ethylenoxid und gegebenenfalls Alkylenoxiden wie Propylenoxid, Butylenoxid, Styroloxid, Epichlorhydrin oder Gemischen dieser Alkylenoxide hergestellt.

Bevorzugt sind mehr als zweifunktionelle Polyether, z. B. mindestens trifunktionelle Ether, da sie kovalent vernetzte Gele ergeben.

Weitere Ausgangsverbindungen für die NCO-Prepolymere bzw. Semiprepolymere sind aliphatische, cycloaliphatische, araliphatische, aromatische oder heterocyclische Polyisocyanate, wie sie z. B. von W. Siefken, Liebigs Annalen der Chemie, Band 562, Seiten 75-136, beschrieben werden.

Als Polyisocyanate kommen beispielsweise die in den DE-A-2 347 299 und 2 521 277 beschriebenen Polyisocyanate in Betracht. Beispiele sind die Toluylendiisocyanate, die Diphenylmethan-diisocyanate in Form ihrer 4,4'- und/oder 2,4'- und/oder 2,2'-Isomeren, ferner Gemische der Diphenylmethandiisocyanate mit ihren drei- und höherkernigen Homologen und Isomeren. Geeignet sind auch die üblichen Abwandlungsprodukte dieser Toluylen- bzw. Diphenylmethan-di- und -poly-isocyanate durch Allophanatisierung, Biuretisierung, Dimerisierung, Trimerisierung, Carbodiimidisierung oder Umsetzung mit stöchiometrisch unterschüssigen Mengen an di- und/oder polyfunktionellen Verbindungen wie Wasser und Di- oder Polyolen.

Ganz besonders bevorzugt sind die Toluylendiisocyanate als 2,4- oder 2,6-Isomere oder ihre Isomerengemische. Aliphatische oder cycloaliphatische Di- und Polyisocyanate können eingesetzt werden, z. B. Hexamethylen-1,6-diisocyanat, Isophorondiisocyanat, biuretisiertes Hexamethylendiisocyanat oder Dicyclohexylmethandiisocyanate in ihren Stellungs- und/oder Stereoisomeren oder deren Gemischen.

Die Menge der bei der Herstellung der erfindungsgemäßen Zusammensetzung einzusetzenden NCO-Prepolymere (entsprechend dem Polyurethan-Feststoff) beträgt 5 bis 30 Gew.-%, vorzugsweise 10 bis 25 Gew.-%, besonders bevorzugt 15 bis 20 Gew.-%, bezogen auf das Gesamtgewicht des Reaktionsgemisch zur Herstellung der Zusammensetzung.

Das bei der Herstellung der erfindungsgemäßen Zusammensetzung mitzuverwendende Wasser erfüllt neben seiner Funktion als Dispersionsmittel für die Zellen, für die übrigen Stoffe und für die Prepolymere bzw. Semiprepolymere zusätzlich auch die Rolle des Reaktionspartners für die Isocyanatgruppen aufweisenden Prepolymere. Der Gehalt an Wasser beträgt 20 bis 90 Gew.-%, vorzugsweise 40 bis 85 Gew.-%, besonders bevorzugt 50 bis 85 Gew.-%, bezogen auf das Gesamtgewicht des Reaktionsgemisches bei der Herstellung der Zusammensetzung.

Als niedermolekulare Kettenverlängerungsmittel bzw. Vernetzer können gegebenenfalls auch Di- und/oder Polyamine mitverwendet werden. Beispiele für solche aliphatischen, cycloaliphatischen oder aromatischen Di- oder Polyamine sind: Ethylendiamin, Hexamethylendiamin, Diethylentriamin, Hydrazin, Guanidincarbonat, N,N'-Diisopropylhexamethylendiamin, 1,3-Bisaminomethyl-benzol, N,N'-Bis-(3-aminopropyl)-ethylendiamin, N,N'-Bis-(2-aminopropyl)-ethylendiamin, N,N'-Bis-(2-aminoethyl)-ethylendiamin, 4,4'-Diamino-diphenylmethan, 4,4'-Dimethylamino-3,3'-dimethyl-diphenylmethan, 2,4'-Diamino-diphenylmethan sowie 2,4- bzw. 2,6-Diaminotoluol.

Der Gehalt an Di- bzw. Polyaminen beträgt 0,2 bis 5 %, bevorzugt 1 bis 4 %, bezogen auf das Gesamtgewicht des Reaktionsgemisches bei der Herstellung der Zusammensetzung.

Als Zusatzstoffe können den erfindungsgemäßen Trägermaterialien dichteerhöhende Substanzen, wie Schwerspat, Metallpulver, Gummigranulat, Tonmehl, Bimsmehl, Glasmehl, Mehl aus Kernen und Schalen von Oliven, Nüssen oder Gesteinsmehl; dichteverringernde Substanzen, wie Polystyrolkügelchen, Holzmehl, Mehl aus Kunststoffabfällen, Mikrohohlperlen, Polyethylenschaumflocken; färbende Agentien wie Pigmente, Farbstoffe; Kurzfasern organischer oder anorganischer Basis wie Glasfasern sowie gelbildende makromolekulare Substanzen wie Cellulosetypen, Alginate, Stärke, Carrageenane zugefügt werden.

Die Herstellung der erfindungsgemäßen biologisch aktiven Zusammensetzungen kann auf verschiedene Weise erfolgen. Man kann z. B. alle Komponenten, d.h. Gelbildner, Polymere mit kationischen Gruppen, oberflächenaktive Kohle, Wasser und Zellen auf einmal zusammengeben und intensiv vermischen. Man kann aber auch die Komponenten nacheinander zusammengeben. Bei der mehrstufigen Arbeitsweise wird das NCO-Prepolymer bzw. -Semiprepolymer zunächst mit einem Teil des Wassers vermischt; dann werden in diese Mischung die mit dem restlichen Wasser aufgeschlämmten Zellen eingerührt. Gemäß einer technisch besonders vorteilhaften Variante dieses Mehrstufenverfahrens wird die gelbildende Substanz zunächst intensiv mit Wasser vermischt und dann die Zellensuspension zugemischt. Eine weitere erfindungsgemäß bevorzugte Verfahrensweise mit mehreren Stufen besteht darin, daß man zunächst feinteilige Kohle und Polymere mit kationischen Gruppen sowie Prepolymere in Wasser gemeinsam vermischt und in ein Gel überführt. Anschließend wird in weiteren Verfahrensschritten das erhaltene Gel in Form von kleinen Partikel mit wachstumsfähige Zellen enthaltende Dispersionen oder Schlämme für 1 bis 50 Stunden vermischt und dann die erhaltene biologisch aktive Zusammensetzung von überschüssigem Schlamm bzw. Dispersion abgetrennt.

Bei diesen Verfahrensweisen kann die Förderung, Dosierung und Mischung der Einzelkomponenten oder Komponentengemische mit den für den Fachmann an sich bekannten Vorrichtungen erfolgen. Die Förderung und Dosierung der Zellensuspension ist z. B. mit einer geeigneten Schnecke möglich. Die anschließende Zumischung der Zellensuspension zum reaktiven, gelbildenden Gemisch wird z. B. in einer Rührwerksmischkammer oder Statikmischer durchgeführt. Dem gelfähigen Reaktionsgemisch kann Luft beziehungsweise Stickstoff zugemischt werden, um eine schaumartige Gelmasse zu erhalten. Ein Schaumgel kann auch durch weitere Maßnahmen, wie z. B. Temperaturerhöhung der Wasserkomponenten oder Konzentrationserhöhung der Prepolymer-Komponente hergestellt werden. Durch Mitverwendung von zellöffnenden Substanzen im Reaktionsgemisch lassen sich die geschäumten Gelmassen vollständig offenzellig herstellen. Als solche Zellöffner kommen beispielsweise hoch ethylenoxidhaltige Polyether mit mindestens trifunktionellen Startermolekülen infrage. Die Menge der Zellöffner beträgt 1 - 15 Gew.-%, bezogen auf das Gesamtgewicht der Reaktionsmischung zur Herstellung der Zusammensetzung.

Die Herstellung der erfindungsgemäßen Zusammensetzungen kann kontinuierlich oder diskontinuierlich vorgenommen werden. Die Arbeitsweise hängt von der Form ab, die man den erfindungsgemäßen Materialien geben möchte. Wenn man z. B. Blöcke oder Stangen herstellen will, die dann in dünne Platten zerschnitten werden sollen, ist vorzugsweise die diskontinuierliche Arbeitsweise durchzuführen. Sollen die erfindungsgemäßen Zusammensetzungen jedoch z. B. in dünnen Stücken geeigneter Abmessungen hergestellt werden, dann ist die kontinuierliche Verfahrensweise vorteilhafter. In diesem Fall produziert man zunächst eine endlose Bandware, die anschließend in einzelne Stücke, z. B. Platten, Folien oder kleine Partikel zerteilt wird. Bei dieser Verfahrensweise kann das reaktionsfähige zellenhaltige Gelgemisch auch, bevor es durch die Reaktion erstarrt, gesprüht oder gerakelt werden. Hierbei kann das reaktionsfähige Gemisch auf die verschiedenartigen Materialien auf Basis von natürlichen oder synthetischen Rohstoffen aufgebracht werden, z. B. auf Folien, Matten, Vliese, Gewirke, Gestricke, Gewebe oder Schaumfolien.

Die erfindungsgemäßen Zusammensetzungen können in den verschiedensten Formen, z. B. als Granulat, Platte, Folie, Block oder Band, eingesetzt werden. Die Anwendung geschieht nach den in der Abwassertechnik üblichen Verfahrensweisen unter aeroben oder anaeroben Bedingungen.

Ein wesentlicher Vorteil der erfindungsgemäßen Zusammensetzungen gegenüber den aus der deutschen Offenlegungsschrift 2 929 872 bekannten, ganze Zellen enthaltenden gelförmigen oder geschäumten Biokatalysatoren auf Basis von Polyurethan liegt in einer verbesserten Abbauleistung von Industrie-Abwässern, die mit organischen Verbindungen beladen sind. Die verbesserte Wirksamkeit der erfindungsgemäßen Zusammensetzungen zeigt sich insbesondere bei Industrie-Abwässern, die z. B. Nitro-, Chlor- und Aminosubstituierte aromatische Verbindungen enthalten.

Ein weiterer Vorteil der neuen Zusammensetzungen ist, daß in ihnen auch empfindliche ganze Zellen eingebettet werden können, wenn als Gelbildner auch Isocyanat-Prepolymere mit geringem Gehalt an reaktiven Isocyanatgruppen eingesetzt werden. Die Wachstumsfähigkeit von gegenüber reaktiven Isocyanaten empfindlichen ganzen Zellen bleibt auf diese Weise erhalten.

Die folgenden Beispiele erläutern die vorliegende Erfindung. Bei den in den Beispielen angegebenen Teilen handelt es sich um Gewichtsteile, sofern nicht anders angegeben ist.

**Beispiel 1**

*a) Herstellung der biologisch aktiven Zusammensetzung*

7 Teile einer wäßrigen Suspension (≈ Biofeuchtmasse), die 15 Gew.-% nach der EP-A-99 020 (veröffentlicht am 25/1/84), Beispiel 1, speziell gezüchteter Zellen enthält, wurden in 67,5 Teile mit Phosphatpuffer auf pH = 7 eingestelltes Wasser eingerührt. Hierzu wurden innerhalb von 30 Sekunden nacheinander 7,5 Teile neutrale feinpulvrige Aktivkohle und 1 Teil pulverförmiges Ionenaustauscherharz mit kationischen Gruppen (Typ: Lewasorb A 50®, Bayer AG) sowie schließlich 17 Teile des Isocyanat-Prepolymers A, dessen Herstellung nachstehend beschrieben ist, unter intensivem Rühren zugefügt. Nach weiteren 45 Sekunden Rührzeit erfolgte die Gelbildung. Man erhielt eine elastische Gelmasse, die anschließend zu Partikeln mit einem mittleren Durchmesser von 2-3 mm zerkleinert wurde.

Das verwendete Isocyanat-Prepolymer A wurde wie folgt erhalten:

Eine Mischung aus 159 Teilen Toluylendiisocyanat (80 % 2,4- und 20 % 2,6-Isomeres) und 1200 Teilen eines trifunktionellen Polyethers, der durch Anlagerung von 60 Gew.-% Ethylenoxid und 40 Gew.-% Propylenoxid an Glycerin erhalten worden ist und eine Hydroxylzahl von 28 besitzt, wird unter Rühren innerhalb von 30 Minuten auf 80°C erwärmt. Bei dieser Temperatur wird die Reaktionsmischung weitere 3 Stunden gerührt und dann auf Raumtemperatur abgekühlt. Das erhaltene Prepolymer hat einen Isocyanatgehalt von 3,7 % und eine Viskosität von 9500 mPa.s bei 25°C.

*b) Reinigung von Abwasser mit der biologisch aktiven Zusammensetzung*

Abwasser, das als organische Verbindung neben Naphthalinsulfonsäuren auch Nitro-, Chlor- und Amino-substituierte aromatische Verbindungen in Konzentrationen bis zu 200 ppm enthielt, wurde mit den nach Beispiel 1a) erhaltenen Zusammensetzungen behandelt. Das Abwasser wurde täglich gewechselt. Nach einmonatiger Versuchsdauer betrug die Abbauleistung der biologisch aktiven Zusammensetzung 98 % des organischen Kohlenstoffs (TOC).

**Beispiel 2**

Die biologisch aktive Zusammensetzung wurde gemäß der Verfahrensweise Beispiel 1 hergestellt. Statt 1 Teil Ionenaustauscher-Pulver wurden 2 Teile einer 26 Gew.-%-igen wäßrigen Lösung eines Polyelektrolyten auf Basis eines Polyaminocarbonsäureesters mit kationischen Gruppen (Typ: Praestol® 185 K, Fa. Stockhausen) verwendet.

Die Abbauleistung der Zusammensetzung wurde gemäß der Verfahrensweise Beispiel 1 mit dem darin beschriebenen Abwassertyp geprüft. Nach ebenfalls einmonatiger Versuchsdauer erreichte die Zusammensetzung einen Abbau des organischen Kohlenstoffs von 92 %.

**Bespiel 3**

*a) Herstellung der biologisch aktiven Zusamensetzung*

In 59 Teilen Wasser wurden 10 Teile neutrale feinpulvrige Aktivkohle (Typ: Carboraffin® AP, Fa. Bayer) und 2 Teile pulverförmiges Ionenaustauschharz auf Polystyrolbasis mit kationischen Gruppen (Typ: Lewasorb® A 50, Fa. Bayer AG) dispergiert und sodann 7 Teile eines Polyethers, der durch Anlagerung von 70 Gew.-%
Ethylenoxid und 30 Gew.-% Propylenoxid an
Trimethylolpropan erhalten worden war und eine
Hydroxylzahl von 56 aufwies, und 22 Teile des in
Beispiel 1 beschriebenen Isocyanat-Prepolymers
zugefügt und unter Stickstoffeinleitung verrührt.
110 Sekunden nach dem Zufügen des
Prepolymers erfolgte die Gelbildung. Man erhielt
eine geschäumte elastische Gelmasse, die in
Partikel von 3-4 Millimeter Durchmesser
zerkleinert wurde.

In einem 1 Liter-Gefäß mit Rühreinrichtung und
Zu- und Ablauf sowie Gasmessvorrichtung
wurden 500 Milliliter Abwasser aus der
Chlorbleicherei einer Zellstoff-Fabrik (Gehalt an
TOC 900 ppm), 30 Milliliter Faulschlamm
(Trockensubstanzgehalt 20 Teile/Liter) aus dem
Faulturm einer kommunalen Kläranlage und 30
Teile der obigen Gelpartikel gegeben und für 24
Stunden bei einer Temperatur von 35° C unter
Sauerstoffausschluß gerührt. Dann wurde unter
Stickstoffbegasung der überschüssige
Faulschlamm mit Abwasser herausgespült. Man
erhielt Gelpartikel, die wachstumsfähige
Mikroorganismen aufwiesen.

*b) Reinigung des Abwassers mit der biologisch
aktiven Zusammensetzung*
30 Teile des nach Beispiel 3a erhaltenen
biologisch aktiven Trägermaterials wurden in ein
in Beispiel 3a beschriebenes Gefäß gegeben und
500 Milliliter Abwasser aus der Chlorbleicherei
einer Zellstofffabrik zugefügt. Das Abwasser wies
einen Gehalt an organischem Kohlenstoff (TOC)
von 900 ppm auf. Die Mischung aus Abwasser
und Trägermaterial wurde unter
Sauerstoffausschluß mit 50 Umdrehungen pro
Minute gerührt. Während der Versuchsdauer
wurde jeweils täglich 50 Milliliter des
behandelten Abwassers entnommen und gegen
50 Milliliter neues Abwasser ausgetauscht. Nach
3 Wochen ab Versuchsbeginn wurden folgende
Werte erhalten: Organischer Kohlenstoff (TOC)
im behandelten Abwasser: 366 ppm.
Biogasproduktion ($CH_4$ + $CO_2$): 67 Milliliter pro
Tag.

**Beispiel 4**

*a) Herstellung der biologisch aktiven
Zusammensetzung*
14 Teile neutrale feinpulvrige Aktivkohle (Typ:
Carboraffin® AP, Fa. Bayer AG) und 4 Teile eines
Polyaminocarbonsäureesters mit kationischen
Gruppen (Typ: Praestol® 185 K, Fa. Stockhausen)
wurden in 152 Teile Wasser dispergiert. Danach
wurden 30 Teile des unter Beispiel 1
beschriebenen Isocyanat-Prepolymers
zugemischt. Nach 90 Sekunden erfolgte die
Gelbildung.
Die erhaltene Gelmasse wurde in Partikel von
2-3 Millimeter Durchmesser zerteilt. 160 Teile
dieser Partikel füllte man in einen Up-Flow-Reaktor von 1,6 Liter Inhalt und fügte 120 Milliliter
Faulschlamm (Trockensubstanzgehalt 7,6 Teile
pro Liter) aus dem Faulturm einer kommunalen
Kläranlage sowie 1 Liter Brüdenkondensat hinzu.
Das Brüdenkondensat, das beim Eindampfen der
Kochlauge in der Zellstoffabrikation erhalten
worden war, wies einen CSB-Gehalt von 44.000
ppm auf und wurde mit Wasser auf 4.900 ppm
verdünnt. Nach einer Rührzeit von 24 Stunden bei
einer Temperatur von 35° C unter Luftausschluß
erhielt man biologisch aktives Trägermaterial.

*b) Reinigung von Abwasser*
In einem 1,6 Liter Up-Flow-Reaktor wurden 160
Teile des unter Beispiel 4a erhaltenen
Trägermaterials eingefüllt und das in Beispiel 4a
beschriebene Brüdenkondensat mit Hilfe einer
Dosierpumpe unter anaeroben Bedingungen
kontinuierlich zu- und abgeführt, wobei die
Durchflussmenge an Abwasser 640 Milliliter pro
Tag betrug. Nach 43 Tagen kontinuierlichen
Betriebs wurde folgender Wert im ablaufenden
Abwasser gefunden: CSB-Wert: 3650 ppm.

**Beispiel 5**

*a) Herstellung der biologisch aktiven
Zusammensetzung*
15 Teile neutrale feinpulvrige Aktivkohle (Typ:
Carboraffin® AP, Fa. Bayer AG) und 4 Teile
pulverförmiges Ionenaustauschharz auf
Polystyrolbasis mit kationischen Gruppen (Typ:
Lewasorb® A 50, Fa. Bayer AG) wurden in 131
Teile einer Belebtschlamm-Suspension (Gehalt
an Trockensubstanz 2 Teile pro Liter), die der
Kläranlage einer Kokerei entnommen war,
dispergiert. Zu dieser Suspension wurden 50
Teile des nachfolgend beschriebenen Isocyanat-
Prepolymers B zugefügt und intensiv vermischt. 2
Minuten nach dem Zufügen des Prepolymers
bildete sich ein Gel. Die Gelmasse wurde in
Partikel von 2-4 Millimeter zerteilt.
Das verwendete Prepolymer B wurde wie folgt
erhalten:
Eine Mischung aus 213 Teilen
Toluylendiisocyanat (80 % 2,4- und 20 % 2,6-
Isomeres) und 1587 Teilen eines Polyethers, der
durch Anlagerung von 60 Gew.-% Ethylenoxid
und 40 Gew.-% Propylenoxid an Glyzerin erhalten
worden war und eine Hydroxylzahl von 28 besitzt,
wurde unter Rühren innerhalb von 30 Minuten auf
80° C erwärmt. Bei dieser Temperatur wurde die
Reaktionsmischung weitere 5 Stunden gerührt.
Dann destillierte man überschüssiges
monomeres Toluylendiisocyanat ab. Das
erhaltene Prepolymer hatte einen
Isocyanatgehalt von 1,9 % und eine Viskosität
von 12960 mPas bei 25° C.

*b) Reinigung von Abwasser*
In eine 3 Liter-OECD-Anlage, die mit Zu- und
Ablauf, Belüftungs- und
Nachklärungsvorrichtungen ausgestattet war, wurden 150 Teile des nach Beispiel 5a hergestellten Trägermaterials gegeben und unter aeroben Bedingungen Kokereiabwasser, das mit Wasser verdünnt worden war und danach einen Gehalt an CSB von 961 ppm und einen Gehalt an Phenolen von 293 ppm aufwies, kontinuierlich zu- und abgeführt. Die pro Tag durchgeleitete Menge an Abwasser betrug 5,8 Liter. Nach einer Laufzeit der Anlage von 4 Tagen wurden folgende Werte im ablaufenden Abwasser gemessen:

CSB-Gehalt: 385 ppm
Gehalt an Phenolen: 162 ppm

### Beispiel 8

*a) Herstellung der biologisch aktiven Zusammensetzung*

900 Teile neutrale feinpulvrige Aktivkohle (Typ: Carboraffin® Ap, Bayer AG) und 200 Teile Ionenaustauschharz auf Polystyrolbasis mit kationischen Gruppen (Typ: Lewasorb® A 50, Bayer AG) wurden in 7400 Teilen Wasser dispergiert. Diese Dispersion wurde mit dem nachfolgend beschriebenen Isocyanat-Prepolymer C im Verhältnis 5 Teile Dispersion zu 1 Teil Prepolymer C kontinuierlich vermischt. Das flüssige Reaktionsgemisch füllte man in jeweils 1 Meter mal 0,2 Meter große Kästen bis zu jeweils 3 Zentimeter Höhe. Nach 3 Minuten erhielt man eine elastische Gelmasse, die anschließend in Partikel von 3 bis 5 Millimeter Durchmesser zerteilt wurde. 3600 Teile dieser Partikel füllte man in einen Up-Flow-Reaktor von 33 Liter Inhalt und fügte 2 Liter Faulschlamm (Gehalt an Trockensubstanz 40 Teile pro Liter) aus der anaeroben Abwasser-Anlage einer Zuckerfabrik sowie 25 Liter Abwasser aus dem Becken der mechanischen Vorklärung einer kommunalen Abwasser-Anlage hinzu. Nach einer Stickstoff-Umwälzung von 12 Stunden bei einer Temperatur von 35° C erhielt man biologisch aktives Trägermaterial.

Das verwendete Prepolymer C war wie folgt erhalten worden:

865 Teile eines Polyethers, der durch Anlagerung von 60 Gew.-% Ethylenoxid und 40 Gew.-% Propylenoxid an Glycerin erhalten worden ist und eine Hydroxylzahl von 28 aufweist, wenn mit 135 Teilen 1,6-Hexamethylendiisocyanat homogen vermischt und auf 105° C erwärmt. Die Reaktionsmischung wird bei dieser Temperatur 7 Stunden gerührt und dann auf Raumtemperatur abgekühlt. Danach wird 0,1 Gew.-% Benzoylchlorid zugefügt und homogen verrührt. Das erhaltene Prepolymer weist einen Isocyanatgruppengehalt von 5,1 Gew.-% und eine Viskosität (bei 23° C) von 7400 mPas auf.

*b) Reinigung von Abwasser*

Das nach Beispiel 6a hergestellte Trägermaterial wurde in dem Up-Flow-Reaktor (33 Liter Inhalt) zur Reinigung von Abwasser unter kontinuierlichen und anaeroben Bedingungen in folgender Weise verwendet:

Es wurden 70 Liter pro Tag von mechanisch vorgeklärtem Abwasser aus einer kommunalen Kläranlage mit einem Gehalt an CSB von 266 ppm und einem Gehalt an TOC von 62 ppm durch den Reaktor geleitet. Nach 6 Tagen kontinuierlicher Betriebsdauer wurden im Ablaufwasser 44 ppm CSB und 15 ppm TOC gemessen.

### Patentansprüche

1. Biologisch aktive Zusammensetzung auf Basis von oberflächenaktiver Kohle, Polymere mit kationischen Gruppen und Zellen mit enzymatischer Aktivität enthaltender Hydrogele, dadurch gekennzeichnet, daß die mittels Polyurethan-Hydrogelen immobilisierten Zellen wachstumsfähige Zellen sind.

2. Zusammensetzung gemäß Anspruch 1, dadurch gekennzeichnet, daß als Polymere mit kationischen Gruppen Ionenaustauschharze mit kationischen Gruppen und als oberflächenaktive Kohle Aktivkohlen dienen.

3. Zusammensetzung gemäß Anspruch 1, dadurch gekennzeichnet, daß als Polymere mit kationischen Gruppen Polymere mit Strukturen, die positiv geladene Stickstoffatome enthalten, und als oberflächenaktive Kohle Aktivkohle dienen.

4. Zusammensetzung gemäß Ansprüche 1 bis 3, dadurch gekennzeichnet, daß als wachstumsfähige Zellen die im Faul- und Belebtschlamm von Kläranlagen vorkommenden Zellen und/oder zur Aktivitätserhöhung speziell adaptierte Belebtschlamm-Zellen dienen.

5. Zusammensetzung gemäß Ansprüche 1 bis 4, dadurch gekennzeichnet, daß die Polyurethan-Hydrogele auf Basis von Toluylendiisocyanat-Prepolymere hergestellt sind, deren Gehalt an Isocyanatgruppen 1,5 bis 4 Gew.-% und deren Gehalt an freiem Toluylendiisocyanat weniger als 1 Gew.-%, bezogen auf das Gewicht des Prepolymers, betragen.

6. Verwendung von Polyurethan-Hydrogele, die oberflächenaktive Kohle und Polymere mit kationischen Gruppen enthalten, als Trägermaterial für immobilisierte, wachstumsfähige Zellen zur Mineralisierung von organischen Inhaltsstoffen in Abwässern und Abluft.

7. Verfahren zur Herstellung von biologisch aktiven Zusammensetzungen auf Basis von oberflächenaktiver Kohle, Polymere mit kationischen Gruppen und wachstumsfähige Zellen enthaltender Hydrogele, dadurch gekennzeichnet, daß man wachstumsfähige Zellen und Polymere mit kationischen Gruppen und oberflächenaktiver Kohle in Polyurethan-Hydrogele einbettet.

8. Verfahren zur biologischen Abwasserreinigung, dadurch gekennzeichnet, daß mit den Zusammensetzungen nach einem der Ansprüche 1 bis 4 in Kontakt gebracht wird.

## Claims

1. Biologically active composition based on hydrogels containing surface-active coal, polymers having cationic groups and cells having enzymatic activity, characterised in that the cells immobilised by means of polyurethane hydrogels are cells which are capable of growth.

2. Composition according to Claim 1, characterised in that ion exchange resins having cationic groups are used as the polymers having cationic groups and active carbons are used as the surface-active coal.

3. Composition according to Claim 1, characterised in that polymers having structures containing positively charged nitrogen atoms are used as the polymers having cationic groups and active carbon is used as the surface-active coal.

4. Composition according to Claims 1 to 3, characterised in that the cells occurring in the digested and activated sludge of clarification plants and/or activated sludge cells specifically adapted for increasing activity are used as the cells which are capable of growth.

5. Composition according to Claims 1 to 4, characterised in that the polyurethane hydrogels are prepared on the basis of toluylene diisocyanate prepolymers whose content of isocyanate groups is 1.5 to 4 % by weight and whose content of free toluylene diisocyanate is less than 1 % by weight, based on the weight of the prepolymer.

6. Use of polyurethane hydrogels containing surface-active coal and polymers having cationic groups, as carrier material for immobilised cells capable of growth for the mineralisation of organic contents in waste waters and outgoing air.

7. Process for the preparation of biologically active compositions based on hydrogels containing surface-active coal, polymers having cationic groups and cells which are capable of growth, characterised in that cells which are capable of growth and polymers having cationic groups and surface-active coal are embedded in polyurethane hydrogels.

8. Process for the biological purification of waste water, characterised in that it is brought into contact with the compositions according to one of Claims 1 to 4.

## Revendications

1. Composition biologiquement active à base de charbon tensioactif, de polymères dotés de groupes cationiques et d'hydrogels contenant des cellules à activité enzymatique caractérisée en ce que les cellules immobilisées à l'aide d'hydrogels de polyuréthanne sont des cellules capables de se reproduire.

2. Composition selon revendication 1, caractérisée en ce que des résines échangeuses d'ions avec des groupes cationiques servent de polymères dotés de groupes cationiques et que des charbons actifs servent de charbons tensioactifs.

3. Composition selon la revendication 1, caractérisée en ce que des polymères avec des structures contenant des atomes d'azote chargés positivement servent de polymères dotés de groupes cationiques et que du charbon actif sert de charbon tensioactif.

4. Composition selon les revendications 1 à 3, caractérisée en ce que les cellules présentes dans les boues putréfiées et activées de stations de décantation et/ou les cellules de boues activées spécialement destinées à l'accroissement de l'activité servent de cellules capables de se reproduire.

5. Composition selon les revendications 1 à 4, caractérisée en ce que les hydrogels de polyuréthanne sont obtenus à partir de prépolymères de toluylènediisocyanate dont la teneur en groupes isocyanates s'étend de 1,5 à 4 % en poids et la teneur en toluylènediisocyanate libre est inférieure à 1 % en poids, par rapport au poids du prépolymère.

6. Utilisation d'hydrogels de polyuréthanne contenant du charbon tensioactif et des polymères avec des groupes cationiques, en tant que support pour des cellules immobilisées capables de se reproduire en vue de la minéralisation des constituants organiques dans les eaux résiduaires et dans l'air vicié.

7. Procédé de préparation de compositions biologiquement actives à base de charbon tensioactif, de polymères avec des groupes cationiques et d'hydrogels contenant des cellules capables de se reproduire, caractérisé en ce que l'on enrobe des cellules capables de se reproduire, des polymères avec des groupes cationiques et du charbon tensioactif dans des hydrogels de polyuréthanne.

8. Procédé pour l'épuration biologique des eaux résiduaires, caractérisé en ce que le contact s'opère avec les compositions selon l'une des revendications 1 à 4.